(19) Europäisches Patentamt
European Patent Office
Office européen des brevets

(11) EP 0 749 956 B1

(12) **EUROPÄISCHE PATENTSCHRIFT**

(45) Veröffentlichungstag und Bekanntmachung des Hinweises auf die Patenterteilung:
**23.06.1999 Patentblatt 1999/25**

(51) Int. Cl.$^6$: **C07C 255/53**, C07C 253/20

(21) Anmeldenummer: **96106334.4**

(22) Anmeldetag: **23.04.1996**

(54) **Verfahren zur Herstellung von 2-Hydroxybenzonitril**

Process for the preparation of 2-hydroxybenzonitrile

Procédé de préparation de 2-hydroxybenzonitrile

(84) Benannte Vertragsstaaten:
**CH DE FR GB LI**

(30) Priorität: **21.06.1995 DE 19522430**

(43) Veröffentlichungstag der Anmeldung:
**27.12.1996 Patentblatt 1996/52**

(73) Patentinhaber:
**HÜLS AKTIENGESELLSCHAFT**
**45764 Marl (DE)**

(72) Erfinder:
**Kleemiss, Wolfgang, Dr.**
**45721 Haltern (DE)**

(56) Entgegenhaltungen:
**DE-A- 1 568 137         DE-A- 2 020 866**
**DE-A- 2 533 245         GB-A- 710 973**

- **PATENT ABSTRACTS OF JAPAN vol. 13, no. 49 (C-565), 3.Februar 1989 & JP-A-63 243064 (MITSUI TOATSU CHEM INC), 7.Oktober 1988,**

**Beschreibung**

Die vorliegende Erfindung betrifft ein Verfahren zur Herstellung von 2-Hydroxybenzonitril durch Dehydratisierung von 2-Hydroxybenzamid in der Gasphase.

2-Hydroxybenzonitril ist ein wichtiges Zwischenprodukt für die Herstellung von biologisch aktiven Substanzen.

Die Herstellung von 2-Hydroxybenzonitril aus 2-Hydroxybenzamid ist grundsätzlich bekannt. Als wasserentziehende Mittel sind jedoch ausschließlich teure Reagenzien beschrieben, die zudem noch in stöchiometrischen oder überstöchiometrischen Mengen eingesetzt werden müssen.

In der Patentschrift JP 12 301/66 ist z. B. die Umsetzung von 2-Hydroxybenzamid mit Phosphornitridchloriden bei 100 bis 600 °C beschrieben.

Die DE-OS 25 33 245 lehrt die Herstellung von 2-Hydroxybenzonitril durch Umsetzung des Amides mit Phosgen in einem unpolaren Lösemittel. Das Nitril wird dabei in einer Ausbeute von > 90 % erhalten. Abgesehen davon, daß man hier Phosgen in überstöchiometrischen Mengen einsetzen muß, ist die Verwendung von Phosgen in technischen Prozessen aufgrund seiner extremen Giftigkeit mit erheblichen Schwierigkeiten verbunden.

Für die Herstellung von 2-Hydroxybenzonitril aus 2-Hydroxybenzamid ist auch die Verwendung von Thiophosphorsäurediamid (Bull. Soc. Chim. Belg. _86_, 4 (1977)), Thionyldiimidazol (Heterocycles _12_, 1285 (1979)) und Trichlormethylchlorformiat (Tetrahedron Lett. 2203 (1986)) als wasserentziehende Mittel bekannt.

Alle diese Methoden sind nur für eine Präparation im Labormaßstab geeignet, da die benötigten Reagenzien teuer sind und darüber hinaus in stöchiometrischen bzw. überstöchiometrischen Mengen eingesetzt werden müssen.

Ferner ist die katalytische Umsetzung von 2-Hydroxybenzoesäuremethylester mit Ammoniak zu 2-Hydroxybenzonitril bekannt (Izv. Akad. Nauk. Kaz. SSR, Ser. Khim. 1987 (2), 62). Als Katalysator wurde hierbei Borphosphat verwendet. Problematisch ist bei diesem Verfahren die Bildung von Methylamin aus Ammoniak und Methanol, das aus dem Methylester stammt. Außerdem besitzt das 2-Hydroxybenzonitril die Tendenz, bei Temperaturen von > 100 °C in flüssiger Phase irreversibel in ein hochschmelzendes Triazin überzugehen. Das heißt, daß gebildetes 2-Hydroxybenzonitril im heißen Reaktor zum großen Teil zum Triazin weiterreagiert. Die Produktausbeute ist daher schlecht. Außerdem eignet sich diese Fahrweise nicht für ein technisches Verfahren, da sich der Reaktor innerhalb von kurzer Zeit zusetzt.

Der vorliegenden Erfindung lag daher die Aufgabe zugrunde, ein Verfahren bereitzustellen, das es ermöglicht, in einfacher und möglichst wirtschaftlicher Weise aus 2-Hydroxybenzamid in guten Ausbeuten 2-Hydroxybenzonitril herzustellen.

Überraschenderweise wurde gefunden, daß man die Dehydratisierung von 2-Hydroxybenzamid zu 2-Hydroxybenzonitril in der Gasphase mit besonders gutem Erfolg heterogenkatalytisch unter vermindertem Druck durchführen kann. Insbesondere kann dazu ein mit Phosphorsäure getränktes Kieselgel als Katalysator eingesetzt werden. Geeigneterweise wird das mit Phosphorsäure getränkte Kieselgel vor dem Einsatz getrocknet. Als Kieselgel ist hier beispielsweise AF 125 der Kali-Chemie geeignet, es können aber auch andere, von der Spezifikation her vergleichbare Kieselgelqualitäten eingesetzt werden. Die Dehydratisierungsreaktion wird vorzugsweise bei einer Temperatur zwischen 200 und 400 °C durchgeführt. Besonders überraschend war auch, daß die Sekundärreaktion des 2-Hydroxybenzonitrils zum hochschmelzenden Triazin nicht stattfindet, wenn die Reaktion bevorzugt bei einem Druck zwischen 5 und 100 mbar, besonders bevorzugt zwischen 20 und 40 mbar, durchgeführt wird. Vorzugsweise wird die Reaktion bei einer LHSV (Liquid hourly space velocity) zwischen 0,05 und 1 h$^{-1}$, besonders vorzugsweise zwischen 0,1 und 0,5 h$^{-1}$, durchgeführt. Der Rohaustrag aus der Dehydratisierungsreaktion bestand in der Regel neben Reaktionswasser zu mehr als 90 Gew.-% aus 2-Hydroxybenzonitril; reines 2-Hydroxybenzonitril wird geeigneterweise durch eine destillative Aufarbeitung erhalten, zu bevorzugen ist hier eine Vakuumdestillation.

Gegenstand der vorliegenden Erfindung ist daher ein Verfahren zur Herstellung von 2-Hydroxybenzonitril durch Dehydratisierung von 2-Hydroxybenzamid in der Gasphase, wobei die Reaktion heterogenkatalytisch unter vermindertem Druck durchgeführt wird.

Der Katalysator kann für den Einsatz beim erfindungsgemäßen Verfahren kugel- oder perlenförmig, splittförmig, strangförmig, röhrenförmig oder auch monolithisch, beispielsweise wabenförmig, ausgeführt sein. Es besteht aber auch die Möglichkeit, den Katalysator in Pulverform einzusetzen. Im allgemeinen kann ein hier verwendeter Katalysator Aluminiumoxid und/oder Siliziumoxid und/oder Titanoxid und/oder Zirkonoxid und/oder Silikate enthalten. Solche oxidischen Materialien werden dann in der Regel mit einer anorganischen Säure, insbesondere Phosphorsäure, behandelt und thermisch nachbehandelt, vgl. auch Beispiel 1.

Beim erfindungsgemäßen Verfahren wird die Reaktion zweckmäßigerweise in einem Rohrreaktor durchgeführt. Geeigneterweise wird hier die Dehydratisierungsreaktion so durchgeführt, daß der Katalysator, ein mit Phosphorsäure, vorzugsweise mit wäßriger 5 bis 85 Gew.-%iger Phosphorsäure, getränktes Kieselgel, auf eine Temperatur von 200 bis 500 °C, vorzugsweise 300 bis 400 °C, besonders vorzugsweise bei 380 bis 390 °C, zunächst erhitzt wird. Das 2-Hydroxybenzamid kann dann in aufgeschmolzenem Zustand auf den Kontakt gegeben werden, wobei die LHSV zwischen 0,05 und 1 h$^{-1}$, vorzugsweise zwischen 0,1 und 0,5 h$^{-1}$, liegen sollte.

Die Dehydratisierung wird beim erfindungsgemäßen Verfahren unter vermindertem Druck, vorzugsweise bei einem Druck zwischen 5 und 100 mbar, besonders vorzugsweise bei einem Druck zwischen 20 und 40 mbar, durchgeführt. Das so erhältliche Rohprodukt, auch Rohaustrag genannt, wird in der Regel durch eine Destillation, vorzugsweise unter Vakuum, gereinigt, wobei die Destillation ohne eine große thermische Belastung des 2-Hydroxybenzonitrils geschehen sollte, da das Produkt zur Bildung eines hochschmelzenden Triazins neigt.

Das erfindungsgemäße Verfahren liefert nunmehr die Möglichkeit, 2-Hydroxybenzonitril in einfacher und wirtschaftlicher Weise herzustellen.

Die Erfindung wird durch die folgenden Beispiele näher erläutert:

**Beispiel 1:**

Herstellung des Dehydratisierkontaktes:

200 g Kieselgel (AF 125 der Kali-Chemie, Hannover) werden mit 500 g 30 Gew.-%iger Phosphorsäure 2 h bei Raumtemperatur gerührt. Der Feststoff wird abfiltriert und dann bei 110 °C über 30 h getrocknet.

Durchführung der Dehydratisierungsreaktion:

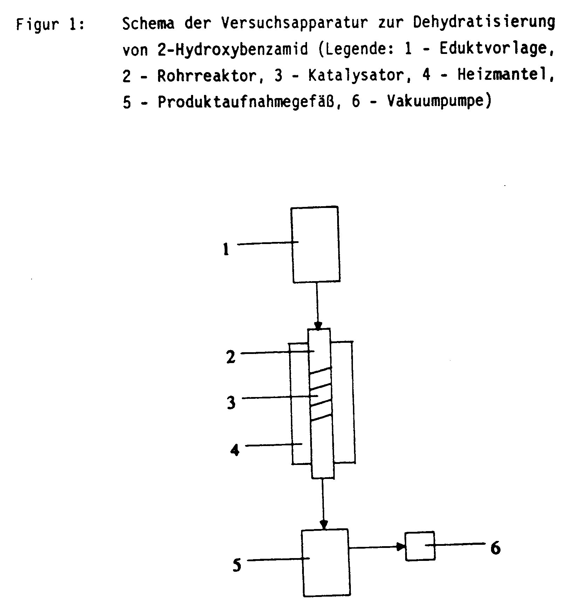

Ein Schema der Versuchsapparatur ist Figur 1 zu entnehmen. 80 g (0,58 mol) 2-Hydroxybenzamid werden in einer beheizbaren Eduktvorlage aufgeschmolzen (Vorlauftemperatur: 150 °C). Bei einem Druck von 30 mbar und einer Katalysatortemperatur von 380 bis 390 °C wird das flüssige Amid auf den Kontakt (Kontaktvolumen: 100 ml) dosiert (ca. 15 ml/h). Die LHSV beträgt 0,15 h$^{-1}$. Als Rohaustrag aus der Versuchsanlage werden 76 g erhalten.

Die destillative Aufarbeitung des Rohaustrages erfolgt in einer gebräuchlichen Destillationsapparatur:

| Fraktion | ST [°C] | KT [°C] | P [mbar] | m [g] | Schmp. [°C] |
|----------|---------|---------|----------|-------|-------------|
| 1 | 60 | - | 0,07 | 9,5 | - |
| 2 | 120 - 173 | 46-173 | 0,01 | 62,3 | 80 - 84 |
| RS | - | - | | 2,4 | > 140 |
| | | | | $\overline{74,2}$ | |

ST = Sumpftemperatur
KT = Kopftemperatur
RS = Rückstand
m = Masse
P = Druck
Schmp. = Schmelzpunkt

Die Fraktion 2 besteht zu 97 Gew.-% aus 2-Hydroxybenzonitril. Damit beträgt die Ausbeute an 2-Hydroxybenzonitril 87 %.

**Beispiel 2:**

210 g (1,53 mol) 2-Hydroxybenzamid werden in einer beheizbaren Vorlage aufgeschmolzen (Vorlauftemperatur: 150 °C). Bei einem Druck von 20 mbar und einer Katalysatortemperatur von 380 bis 400 °C wird das flüssige Amid auf den Kontakt (Kontaktvolumen: 100 ml, Katalysatorherstellung vgl. Beispiel 1) dosiert (ca. 20 ml/h). Die LHSV beträgt 0,2 h$^{-1}$. Als Rohaustrag des Kontaktofens werden 202 g erhalten.

Die Destillation erfolgt in einer einfachen Destillationsapparatur:

| Fraktion | ST [°C] | KT [°C] | P [mbar] | m [g] | Schmp. [°C] |
|----------|---------|---------|----------|-------|-------------|
| 1 | 60 | - | 0,08 | 10 | - |
| 2 | 120 - 175 | 50-172 | 0,1 | 163 | 80 - 85 |
| RS | - | - | - | 20 | - |
| KF | - | - | - | 5 | - |
| | | | | $\overline{198}$ | |

ST = Sumpftemperatur
KT = Kopftemperatur
RS = Rückstand
m = Masse
KF = Kühlfalle
P = Druck
Schmp. = Schmelzpunkt

Die Fraktion 2 besteht zu 97 Gew.-% aus 2-Hydroxybenzonitril. Damit beträgt die Ausbeute an 2-Hydroxybenzonitril 87 %.

Beispiel 3:

150 g (1,09 mol) 2-Hydroxybenzamid werden in einer beheizbaren Vorlage aufgeschmolzen (Vorlauftemperatur: 150 °C). Bei einem Druck von 30 mbar und einer Katalysatortemperatur von 380 bis 390 °C wird das flüssige Amid auf den Kontakt (Kontaktvolumen: 100 ml, Katalysatorherstellung vgl. Beispiel 1) dosiert (ca. 10 ml/h). Die LHSV beträgt 0,1 h$^{-1}$. Als Rohaustrag werden 145 g erhalten.
Die Destillation erfolgt in einer einfachen Destillationsapparatur:

| Fraktion | ST [°C] | KT [°C] | P [mbar] | m [g] | Schmp. [°C] |
|----------|---------|---------|----------|-------|-------------|
| 1 | 65 | - | 0,1 | 6 | - |
| 2 | 125 - 175 | 50-170 | 0,1 | 114 | 79 - 83 |
| RS | - | - | - | 15 | - |
| KF | - | - | - | 5 | - |
| | | | | $\overline{140}$ | |

ST = Sumpftemperatur
KT = Kopftemperatur
RS = Rückstand
m = Masse
KF = Kühlfalle
P = Druck
Schmp. = Schmelzpunkt

Die Fraktion 2 besteht zu 97 Gew.-% aus 2-Hydroxybenzonitril. Damit beträgt die Ausbeute 85 %.

Patentansprüche

1.  Verfahren zur Herstellung von 2-Hydroxybenzonitril durch Dehydratisierung von 2-Hydroxybenzamid in der Gasphase, wobei die Reaktion heterogenkatalytisch unter vermindertem Druck s.Anspr.1 unter vermindertem Druck durchgeführt wird.

2. Verfahren nach Anspruch 1,
dadurch gekennzeichnet,
daß als Katalysator ein mit Phosphorsäure behandeltes Kieselgel eingesetzt wird.

3. Verfahren nach den Ansprüchen 1 und 2,
dadurch gekennzeichnet,
daß das mit Phosphorsäure getränkte Kieselgel vor dem Einsatz getrocknet wird.

4. Verfahren nach den Ansprüchen 1 bis 3,
dadurch gekennzeichnet,
daß die Reaktion bei einer Temperatur zwischen 200 und 400 °C durchgeführt wird.

5. Verfahren nach den Ansprüchen 1 bis 4,
dadurch gekennzeichnet,
daß die Reaktion bei einem Druck zwischen 5 und 100 mbar durchgeführt wird.

6. Verfahren nach Anspruch 5,
dadurch gekennzeichnet,
daß die Reaktion bei einem Druck zwischen 20 und 40 mbar durchgeführt wird.

7. Verfahren nach den Ansprüchen 1 bis 6,
dadurch gekennzeichnet,
daß die Reaktion bei einer LHSV zwischen 0,05 und 1 $h^{-1}$ durchgeführt wird.

8. Verfahren nach Anspruch 7,
dadurch gekennzeichnet,
daß die Reaktion bei einer LHSV zwischen 0,1 und 0,5 $h^{-1}$ durchgeführt wird.

9. Verfahren nach den Ansprüchen 1 bis 8,
dadurch gekennzeichnet,
daß reines 2-Hydroxybenzonitril aus dem Rohaustrag der Dehydratisierung durch eine destillative Aufarbeitung erhalten wird.

## Claims

1. A process for the preparation of 2-hydroxybenzonitrile by dehydration of 2-hydroxybenzamide in the gas phase, the reaction being carried out by heterogeneous catalysis under reduced pressure.

2. A process according to claim 1, characterized in that a silica gel treated with phosphoric acid is used as catalyst.

3. A process according to either of claims 1 and 2, characterized in that the silica gel impregnated with phosphoric acid is dried before use.

4. A process according to any of claims 1 to 3, characterized in that the reaction is carried out at a temperature of from 200 to 400°C.

5. A process according to any of claims 1 to 4, characterized in that the reaction is carried out at a pressure of from 5 to 100 mbar.

6. A process according to claim 5, characterized in that the reaction is carried out at a pressure of from 20 to 40 mbar.

7. A process according to any of claims 1 to 6, characterized in that the reaction is carried out at an LHSV of from 0.05 to 1 $h^{-1}$.

8. A process according to claim 7, characterized in that the reaction is carried out at an LHSV of from 0.1 to 0.5 $h^{-1}$.

9. A process according to any of claims 1 to 8, characterized in that pure 2-hydroxybenzonitrile is obtained from the crude output of the dehydration by work-up by distillation.

## Revendications

1. Procédé de fabrication de 2-hydroxybenzonitrile par déshydratation de 2-hydroxybenzamide en phase gazeuse, dans lequel la réaction est conduite par catalyse hétérogène à pression réduite.

2. Procédé selon la revendication 1,
caractérisé en ce qu'
on utilise comme catalyseur un gel de silice traité avec de l'acide phosphorique.

3. Procédé selon les revendications 1 et 2,
caractérisé en qu'
on sèche avant utilisation le gel de silice imbibé d'acide phosphorique.

4. Procédé selon les revendications 1 à 3,
caractérisé en ce qu'
on conduit la réaction à une température comprise entre 200 et 400°C.

5. Procédé selon les revendications 1 à 4,
caractérisé en ce qu'
on conduit la réaction à une pression comprise entre 5 et 100 mbars.

6. Procédé selon la revendication 5,
caractérisé en ce qu'
on conduit la réaction à une pression comprise entre 20 et 40 mbars.

7. Procédé selon les revendications 1 à 6,
caractérisé en ce qu'
on conduit la réaction à un débit horaire de liquide compris entre 0,05 et 1 $h^{-1}$.

8. Procédé selon la revendication 7,
caractérisé en ce qu'
on conduit la réaction à un débit horaire de liquide compris entre 0,1 et 0,5 $h^{-1}$.

9. Procédé selon les revendications 1 à 8,
caractérisé en ce qu'
on obtient le 2-hydroxybenzonitrile pur par un traitement de distillation à partir de l'extrait brut de la déshydratation.

Figur 1:    Schema der Versuchsapparatur zur Dehydratisierung
            von 2-Hydroxybenzamid (Legende: 1 - Eduktvorlage,
            2 - Rohrreaktor, 3 - Katalysator, 4 - Heizmantel,
            5 - Produktaufnahmegefäß, 6 - Vakuumpumpe)